# EUROPEAN PATENT SPECIFICATION

(11) **EP 0 589 074 B1**
(45) Date of publication and mention of the grant of the patent: **21.11.2001**
(21) Application number: 92116154.3
(22) Date of filing: 22.09.1992
(51) Int. Cl.: A61K 31/70, A61K 33/06

(54) **Carbohydrate complexes for destruction of resistant cancer cells**
Kohlenhydrate-Komplexe zur Vernichtung von Krebszellen
Complexes d'hydrates de carbone pour la destruction de cellules cancéreuses

(43) Date of publication of application: 30.03.1994
(73) Proprietor: Takayanagi, Takeo, Dr., New York, N.Y. 10705 (US)
(72) Inventor: Takayanagi, Takeo, Dr., New York, N.Y. 10705 (US)
(74) Representative: Gille Hrabal Struck Neidlein Prop Roos

(56) References cited:
- EP-A- 0 431 736
- FR-A- 2 126 134
- PATENT ABSTRACTS OF JAPAN vol. 12, no. 122 (C-488)15 April 1988
- PATENT ABSTRACTS OF JAPAN vol. 16, no. 490 (C-994)12 October 1992

## Description

### BACKGROUND OF THE INVENTION :

### 1. Field of the Invention

The present invention relates to novel saccharide complexes for the treatment of resistant cancer.

### 2. Related Art

Cancer has been a fatal disease since the year 300 B.C.

Cancer cells generally constitute a divergent and mixed population, and among said population there are survivor cells (0.1-2%), which are resistant against all forms of treatment. If a cell escapes it will multiply rapidly to a trillion cells in 20 years. Accordingly, cancer seems to be incurable, and chemically resistant cancer cells are not probable.

An effective treatment of cancer cells was described in JP-A- 62 242 658 by the direct combination of a substance with biological activity, like for instance a carcinostatic agent or an antiobiotic agent with a specific azo compound.

Products with cytostatic activity are described in FR-A-2 126 134, such products consisting of cyclophosphamide in combination with another compound, in particular with an inorganic magnesium derivative. The combination displays an improved cytostatic activity.

Cyclodextrins are described in EP-A- 0 431 736 to be suitable for the treatment of diseases related with male hormones, like e.g. the treatment of a prostate enlargement

A composition suitable to prevent colon and rectal cancer has been described in JP-A- 41 79 459, the composition consisting of roasted dextrin with alpha-amylase and/or glycoamylase.

In the present state of affairs it appears that chemical synthesis and the medical treatment of cancer has not developed significantly and hereupon a study of advisable biologically active substances in vitro and in vivo has been undertaken.

During the study of a survivor cell membrane, a series of saccharide complexes that have remarkable anti-cancer abilities has been discovered. The result of the investigation has made it possible to anticipate the achievement of cancer therapy and may be designed to provide a clue to the solution of the problem.

In the present invention the common saccharides serve as the starting material hydrolyzed by acid or an enzyme. Also, commercially available saccharide esters or ethers are used for the same purpose.

The hydrolysis is carried out smoothly in a diluted HCl or H₂SO₄ solution to the extent of 7.5-15% of acid under heating or in an enzymic solution. Primarily, a fundamental criterion of the present hydrolysis type of starch and the harvest separation of ingredients are given in the following table.

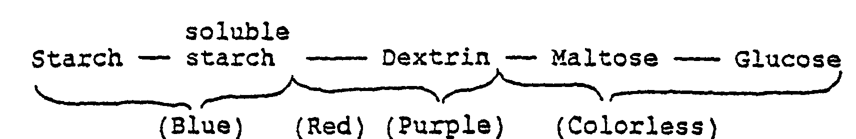

The grades of the hydrolysis and the result of the color reaction with Iodine is partly divided. During the progressing reaction a starch-iodine reaction and microscopic observation of the destruction state of cancer cells should be continuously monitored. The most effective fraction strikes the end of slightly red color parts, and at that time the heating is stopped and the mixture is subjected to a powerful air-drying process. The resulting product is almost viscous.

The resulting product consists of stereospecific products that are extensively effective and do not necessarily separate the fractions.

The hydrolysed saccharide was dissolved in 2-methoxyethanol in the presence of a further component which produces the specific destructing effect on the cancer cells and is characterised hereafter as reinforcing agent or cell destructive factor.
It is necessary according to the invention to add a magnesium compound to the solution of the modified saccharide, such magnesium compound being magnesium sulfate.

It is possible to provide the saccharide by subjecting a carbohydrate compound which is itself only slightly soluble to acetolysis as displayed in the following table.

| The Table of acetolysis | | | | |
|---|---|---|---|---|
| Specimen of saccharides | Composition after acetolysis | | | Heating Times (Decomposition product) |
| | Acetic anhydride | Acetic acid | Sulfuric acid | |
| Cellulose | 10 | 10 | 1 | 30°C, 2 days (Simple saccharide), 5 days (Cellobiose) |
| Dextran | 8 | 5.3 | 1 | 30°C, 7 days after 7 days 80 °C. 30 min. Hirclose |
| " | 8 | 5.3 | 1 | After acetylation 40°C. 30 min. (Trisaccharides) |
| Mannan | 10 | 10 | 1 | After acetylation 40°C. 13 hr. |
| Glycopeptide | 10 | 10 | 1 | 20°C. 1 - 4 days (Simple Polysaccharides) |

Several drops of the mixture are diluted with water. Lugol reagent is added to this solution, and the color reaction and a microscopic test are simultaneously carried out to determinate the most effective part. Saccharide ester is also available for the same purpose. The esterification of saccharides is easily carried out in a usual manner. Should satisfactory hydrolysates be found, the enzymic hydrolysis products may be added by another.

The enzymes play an important role in anaerobic energy production by supplying steric oxidation. When the study of hydrolysis continued, it was confirmed that the cyclodextrin also has a remarkable anticancer ability, and it was first introduced to the present invention.

The cyclodextrin was discovered just over 100 years ago (1891) by Vielliears. It was studied in detail and the following polymorphic forms of steric compounds have been chromatographically isolated and identified.
α-,β-,γ-,∈-,δ-,ζ-,η-,θ- cyclodextrin

These compounds are important as a chemical for the study of host and guest chemistry.

According to the present invention, the preparation of the most chemotherapeutically effective saccharide compounds are accomplished in a solvent, particularly in ethylene glycol monomethyl ether, accompanying a 1-3 destruction factor. The condensation occurs by a magnesium sulfate solution owing to the electrostatic attraction, wherein the included components are indiscriminately in a liquid or a solid state. This was confirmed in all our experiments and a related method has hitherto not been published.

As above described, cancer cells constitute a mixed population, whereas a single effective agent cannot penetrate the significant resistance of the cancer cells. Cancer cells have a remarkable ability to cope with a wide range of external environments. Further, many difficulties arise during cancer therapy and it is most troublesome that there is a total number of 60 species of cells. Naturally a large number of competitors are required.

In general, carbohydrates (saccharide) are accepted as an important mobile carrier of metabolically available energy source in the cells. Indeed, the several hydroxyl groups in the compounds are favorably located and their mechanism accounts for the catalytic effect. The carbohydrates can be hydrolyzed by acid or an enzyme and they provide excellent material for a selected investigation. Furthermore, they are highly acceptable to cyclization (acylation), acetylation, alkylation and esterification in the usual manner.

The applied factors are prepared as follows. The amino compounds and amino acid group are easily obtained by kneading with various metal oxydes adding a small quantity of water as a friction polymer. The diazonium salts of the same are effective and are stabilized in clathrate compounds, or the diazonium salts easily form additional complexes. The metal fixed enzymes are easily applied to the present invention.

Pursuing a solution to the mechanism of resistance of cancer cells to discover the cause of said resistance. The autolysis phenomenon necessitates a small amount of metal salts and from the clue noted in the text the following compounds were provided experimentally:
I(ZnCl₂), Cu(NH₄)₂ Cl₄, I(CuCl₂),
I(CaCl₂) and other metal salts.
Furthermore, as organic substances, the following compounds would occur to be suitable
tert-(CH₃)₃CC₆(OH)₂, C₄H₂N₂O₄,
p-C₆H₄O₂, C₁₀H₆O₂, (CH₃)₃CC₆H₃(OH₃)OH. Since these results suggest the stimulation of an exhaustive screening of the inorganic or organic compounds.

A great number of random screening tests have been effected and the results are present in the following data.
I(ZnCl₂)-NN-CH₂COOC₂H₅, tert-(CH₃)₃CC₆H₃(OH₂)₂ I(CaCl₂)-NN-CH₂COOC₂H₅, I(CuCl₂)-NHC₆H₃(OH) COOH, HNHNOCNH₄C₆, CaNCOOC₂H₅, I(CaCl₂), CaN-CH₂COOC₂H₅, Na₂ CH₂ COOC₂H₅, Cu(NH₄)₂Cl₄, HONHCONH₂, Mg:NCH₂CH₂Br, 1,4-C₁₀H₆O-NCONHCH₂CH₂-OH, HONHCOCH₂C₂NH₂, C₅H₄N-CONHNH₂, 1,4-C₁₀H₆O:N·CH₂COOC₂H₅, p-C₆H₄O-NCH₂COOC₂H₅ · 6-C₅H₃N₄-N:N-CH₂COOC₂H₅, I (CuCl₂) -NN-CH₂COOC₂H₅, Hg:NCH₂CH₂Br, Cu: NCH₂CH₂Cl Hg:NCH₂CH₂SO₃H, -NN-C₆H₃ (OH) COOH, -N: N-OS-NH (CH₂:CHCH₂), C₄H₂N₂O₃:N-COOC₂H₅ 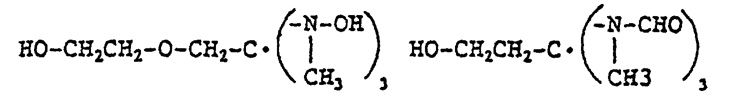 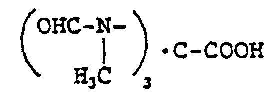 or ester Schweizer reagent, -N:N-CH₂CH₂SO₃H, -N:N-CH₂COOC₂H₅,
(CH₃)₃CC₆H₃(OCH₃)OH, (CH₃)₃CO₆H₃(OH)₂:N:N-CH₂COOC₂H₅.

Chemotherapy may be defined as the selective destruction of pathogenic organisms within a host by chemical agents. The attempt to resolve the cancer problem was unsuccessful. The fate of the drugs was traced by using the following Table.

Cell destruction % after 10Mn. after 5-7 days on an ascites mouse easily goes up to the following

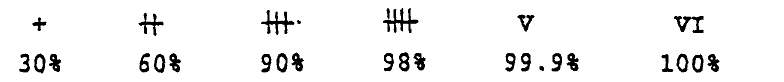

falls under the category of survivor, the survivor invades the tissue.

Even the best drug barely goes to 98% and prolongs life. 99.9% of the mice experience 30% complete recovery after injection. 0.1-0.001% will correspond to strong survivor cells, which should be destroyed in 10 minutes for complete recovery. At present there is no approach to the long standing cancer problem.
At the time of cell destruction, microscopic observation is excellent, forming glittering bubbles and twinkling all over under reflected sunlight. The same phenomenon occurs by using safety match powder.

At last, laboratory synthesis of survivor cell destruction complexes have been achieved by the substitution of several OH functional groups in the saccharide hydrolysates with the following radicals:
NaSH, NaNH₂, NaOCH₃, NaF, NaN₃ and NaBH₄.

These complexes can wipe out the resistant cancer survivor cells thoroughly without reinforcing agents. The disappearance of cancer cells is presumably a result of the increased enzymic function of the saccharide hydrolysate, because a radical change is strengthened by the fact that all molecules have one pair of electrons. This fact may hold the key to the solution of long standing cancer problem.

The saccharide complexes have the advantage that the intended effective agents can be included within the complexes, liquid substances will be pulverized, malodorous active agents will be deodorized, insoluble material will be easily water soluble and have high stability.

Furthermore, it is now substantiated that this invention has the advantage of obtaining many effective substances through adequate variation and combination of the substituents and the condensation components. Finally numerous new effective compounds necessary for combination therapy against resistant viruses have been found.

The new complex-compound of this invention has accurate inhibitory properties against the growth of tissue, therefore the complexes are available for the treatment of malignant tumors or to inhibit new formations. The related experiment has been carried out since the year 1968 and is now under the support of the National Cancer Institute of America.

The compounds of this invention are, as mentioned above, nearly not toxic, tasteless, stimulusless and odorless and furthermore have no side effects and are water soluble. Therefore, this hybrid is widely applicable for clinical use.

In the following examples there are described several preferred embodiments to illustrate the invention.

### Example 1

2% of starch was dissolved in 40 ml of a 7.5% HCl solution for almost 15 min. and heated in a water bath. The time-course of heating was followed with a resulting starch-iodine color reaction and simultaneous observation of cell destruction stated microscopically in the ascites. The most effective part of this case corresponds to the end of the red part in the table indicated. Then the mixture was poured into the water, and was neutralized with barium carbonate and the resulting product was separated. The product was taken out and was dissolved in a 40 ml 2-methoxyethanol mixture with a 1 : 1/2 ratio reinforcing agent selected from the above cited group that is appropriate to the intended cancer species
tert-(CH₃)₃CC₆H₃(OH)₂:N-CH₂COOC₂H₅
Cu:N-CS-NH(CH₂:CH-CH₂)

Then into the mixture was poured the concentrated magnesium solution with vigorous stirring until the solution completely coagulated. The product was washed with petroleum ether and air-dried on filter paper to obtain an easily water soluble complex that decomposed at 180°C. The resulting complex has the following formula.

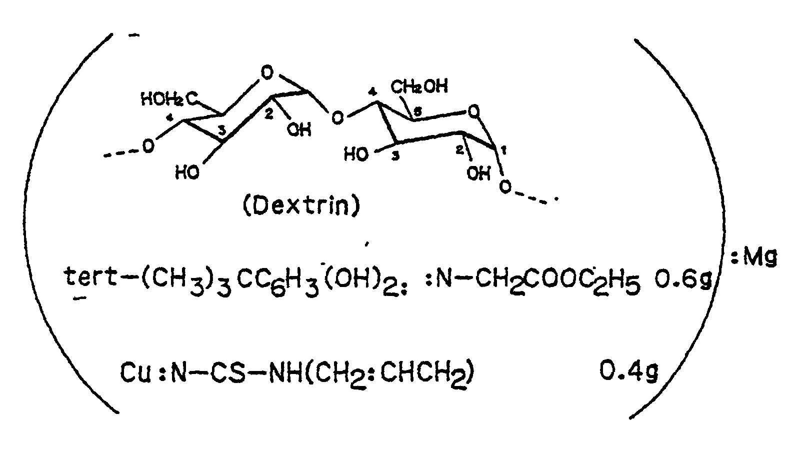

### Example 2

1 g of milk sugar was dissolved in 40 ml of a 7.5% HCl solution about 10 Min. and heated in a water bath. The time of heating may lead to a determination regarding the state of cell destruction. Then the mixture was poured into the water and was neutralized with barium carbonate and the resulting product was separated. The hydrolysates were taken out and the solution was dissolved in 50 ml of 2-methoxyethanol, and the following reinforcing agents were added into the mixture
I(CuCl₂)-NN-C₆H₃(OH) COOH
C₄H₂N₂O₃-N-CH₂COOC₂H₅
Metal fixed pectinase.

Successively undergoes electromagnetic condensation with concentrated MgSO₄ solution. The resulting product was air-dried on a filter paper and has the following structural formula:

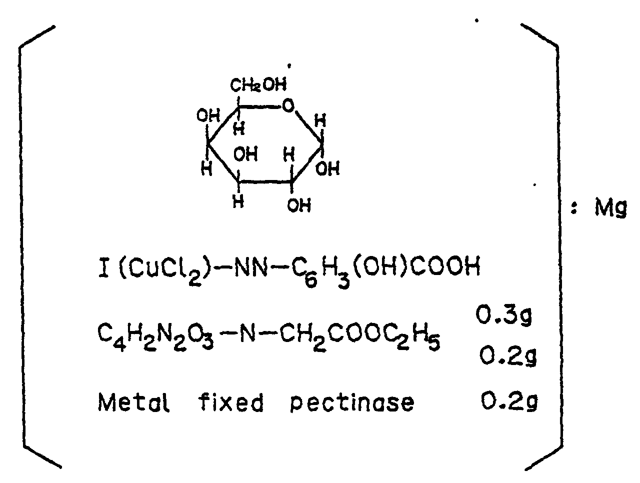

### Example 3

2g of cellulose were added portionwise into the mixture of the following acid:

| | |
|---|---|
| Acetic anhydride | 17 ml |
| Acetic acid | 17 ml |
| Sulfuric acid | 1.8 ml |

and cooled and then the mixture should be allowed to stand 7 days at 25°C-30°C. Finally it should be heated at 80°C for 30 Min.

The mixture was then was poured into the water. It was neutralized with sodium carbonate and the aqueous mixture was extracted with chloroform. The extract was dried under reduced pressure and the resulting acetyl products are subjected to deacetylation in the usual manner. Further manipulation should be done exactly according to the previously outlined procedure. The following formula of the product was subsequently provided.

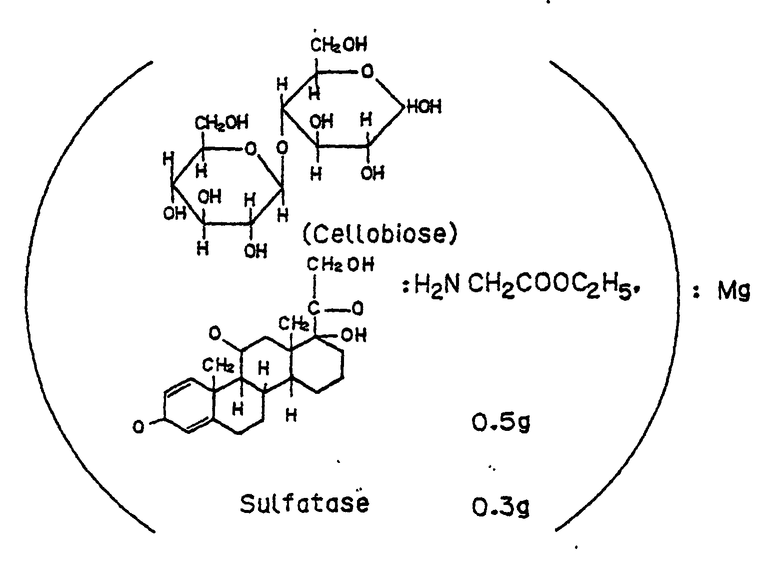

### Example 4

2 g of N-Diethylaminoethyl D-Glucosamine were dissolved in 40 ml of 2-methoxyethanol and then the reinforcing agents
Hg:N-CH₂CH₂SO₃H
Mg:N CH₂CH₂Br 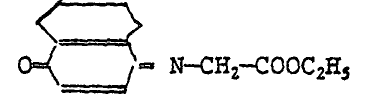 were added to the mixture. Successively, a concentrated magnesium sulfate solution was poured into the mixture until it completely coagulated. The resulting product was taken out and subjected to air-drying on the filter paper to obtain an water soluble complex which decomposed at 180°C and the resulting complex has the following structural formula:

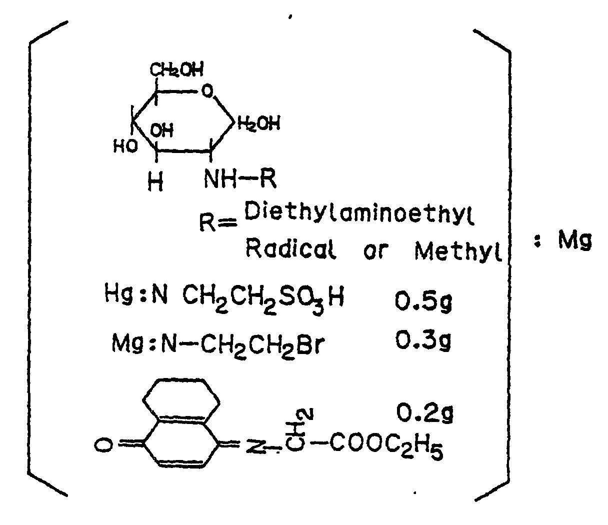

### Example 5

1 g Starch(2.3 µmol) was added to β-Galactosidase, (8.0.unit), 0.05 citric acid puffer solution, pH4.0, 0.6 ml into a moderate quantity of water and the mixture was allowed to stand at 37°C for 15 hr. The product secured comprises various ambiguous steric saccharides.

Then the mixture was filtrated and the reinforcing agent in the ratio of 1 : 1/2 was added into the mixture and it was allowed to stand at room temperature overnight. Then 50 ml 2-methoxyethanol was added into the mixture and successively underwent magnesium condensation. The resulted coagulum is air-dried on filter paper. According to this invention the used enzyme may feasibly vary by changes in experimental conditions. The resulting complex is water soluble and has the following structural formula:

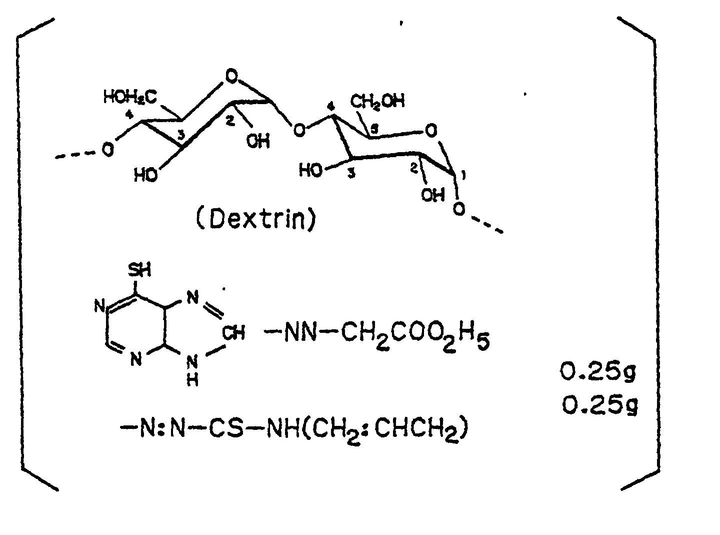

### Example 6

The chemicals α-, β-, γ- cyclodextrin were first introduced in the present invention with extraordinary effect regarding cancer chemotherapy. These compounds are used individually or mixed, moreover with adequate destruction agents in 2-methoxyethanol by condensing agent MgSO₄ coagulation of the solution was brought about.

For preparation of the complex, 2 g β-cyclodextrin was dissolved in 60 ml of 2-methoxyethanol and then as a reinforcing agents

| | |
|---|---|
| C₁₀H₁₂N₅O₄-N:N-CH₂COOC₂H₅ | 0.7 g |
| Hg:N-CH₂CH₂Br | 0.7 g |
| -N:N-CH₂COOC₂H₅ | 0.1 g |

were added to the mixture. Successively, a concentrated magnesium solution was poured into the mixtures until it completely coagulated. The resulting product was taken out and subjected to air-drying, which has the following structural formula:

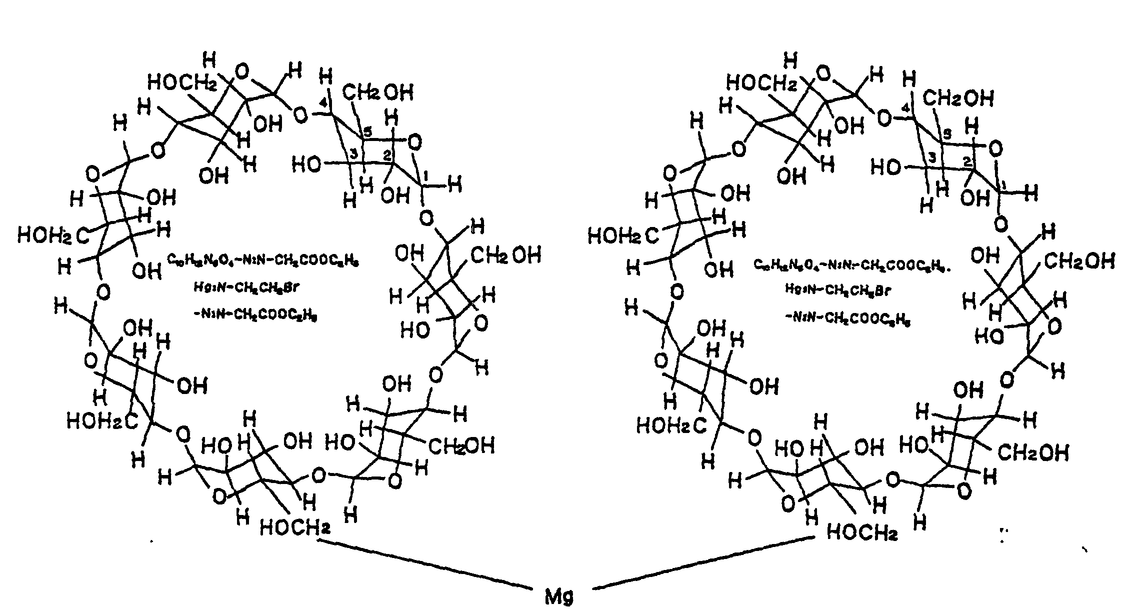

### Example 7

For preparation of the new saccharide complex 2 g of α-, β-, γ- cyclodextrin (individually or mixed) were dissolved in 60 ml methoxyethanol and then the reinforcing agents

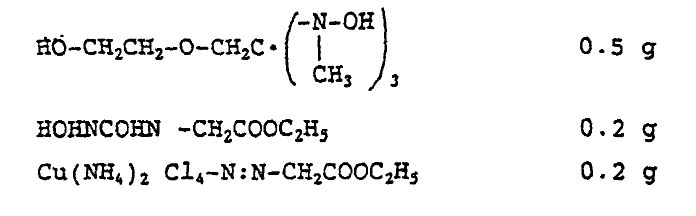

were added to the mixture. Successively, a concentrated magnesium sulfate solution was poured into the mixture until it completely coagulated. The resulting product, which was taken out and subjected to air-drying, has the following structural formula:

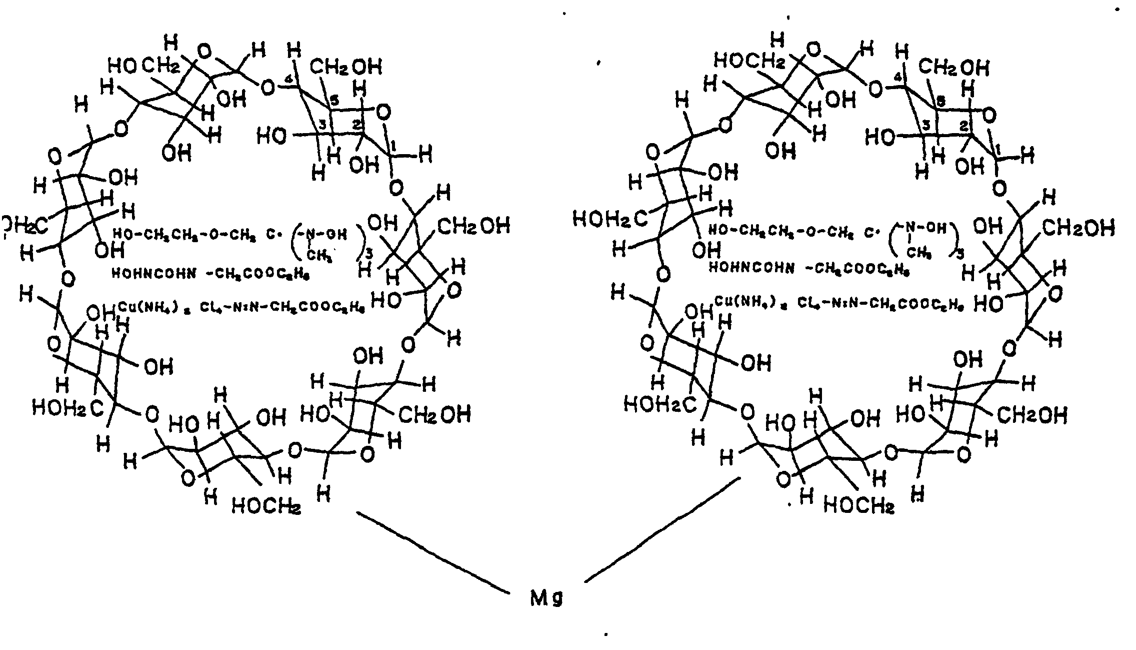

### Example 8

For preparation of the saccharide complex, 2 g of Diethylaminoethyl Cellulose were dissolved in 60 ml 2-methoxyethanol and then the reinforcing agents

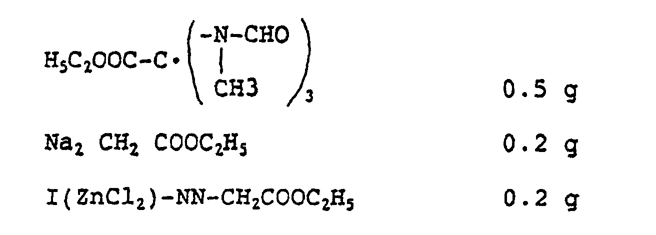

were added to the mixture. Successively, a concentrated magnesium sulfate solution was poured into the mixture until it completely coagulated. The resulting product, which was taken out and subjected to air-drying, has the following structural formula:

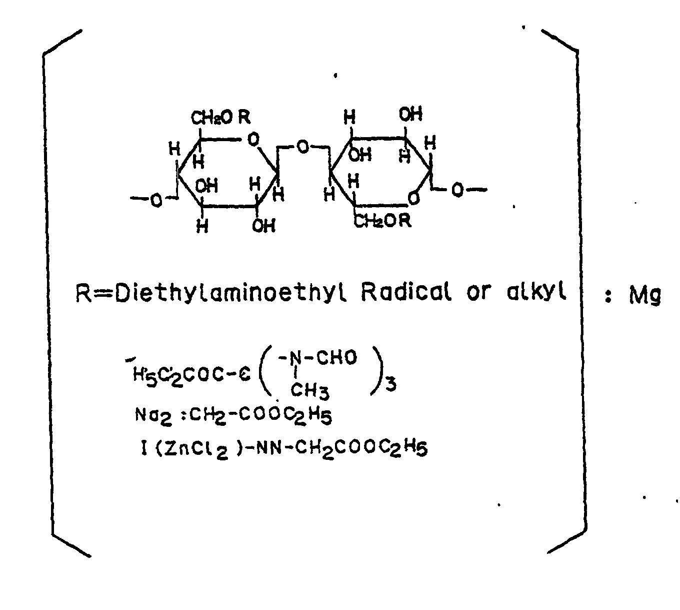

### Example 9

For preparation of the new saccharide complex 2 g of Butyl-cellulose was dissolved in 60 ml 2-methoxyethanol and then the reinforcing agents

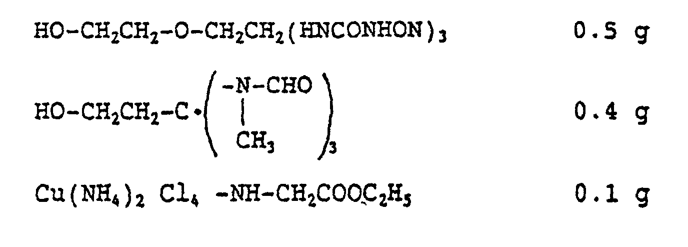

were added to the mixture. Successively, a concentrated magnesium sulfate solution was poured into the mixture until it completely coagulated. The resulting product, which was taken out and subjected to air-drying, has the following structural formula:

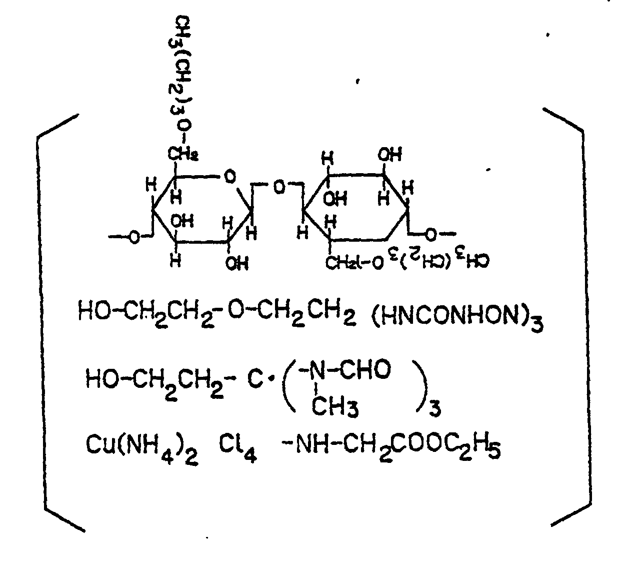

### Example 10

For preparation of the new saccharide complex, 2 g of Diethylaminoethyl-D-glucosamine were dissolved in 60 ml of 2-methoxyethanol and then the cell destruction factors
tert-(CH₃)₃CC₆H₃(OH₂:N-CH₂COOC₂H₅, 0.5 g
Hg:M-CH₂CH₂-SO₃H
-NN-C₇H₅O₃
were added to the mixture. Successively, a concentrated magnesium sulfate solution was poured into the mixture until it completely coagulated. The resulting product, which was taken out and subjected to air-drying, has the following structural formula:

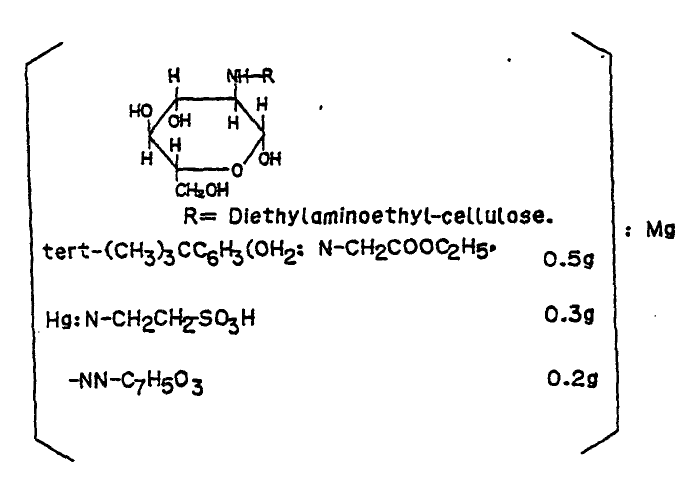

### Example 11

For preparation of the new saccharide complex, 2 g of β-D-Mannose were dissolved in 60 ml 2-methoxyethanol and then the destruction factors
tert-(CH₃)₃CC₆H₃(OH₂:N-CH₂COOC₂H₅,
Hg:N-CH₂CH₂-SO₃H
-NN-C₇H₅O₃
were added to the mixture. Successively, a concentrated magnesium sulfate solution was poured into the mixture until it completely coagulated. The resulting product, which was taken out and subjected to air-drying, has the following structural formula:

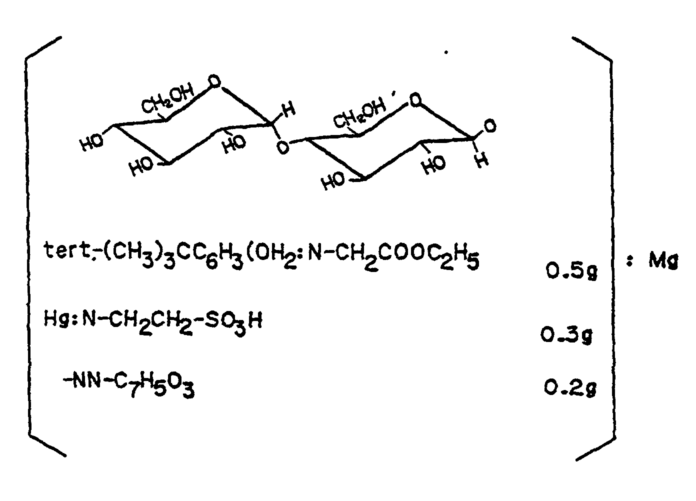

### Example 12

For preparation of the new saccharide complex 2 g of α-, β-, γ-cyclodextrin (individually or mixed) were dissolved in 60 ml of 2-methoxyethanol and then a 20 ml of Hydrogen Sulfide (NaSH) solution added to the mixture. A simultaneous reaction occurs with the rise of temperature, it then becomes cloudy and very soon a flocculent precipitate separates out from the solution. Wherein, the following sodium compounds:
NaNH₂, NaN₃, NaF, Na-OCH₃, NaBH₄
were used instead of NaSH for a similar reaction. A precipitate was dissolved in 60 ml of 2-methoxyethanol, if necessary, and

| | |
|---|---|
| p-C₆H₄O-N-CH₂COOO₂H₅ 0 | 0.6 g |
| HOHN-CO-CH₂CH₂NH₂ | 0.4 g |

added to the mixture and then underwent magnesium sulfate condensation. The resulting coagulum, which dried on a filter paper, has the following structural formula:

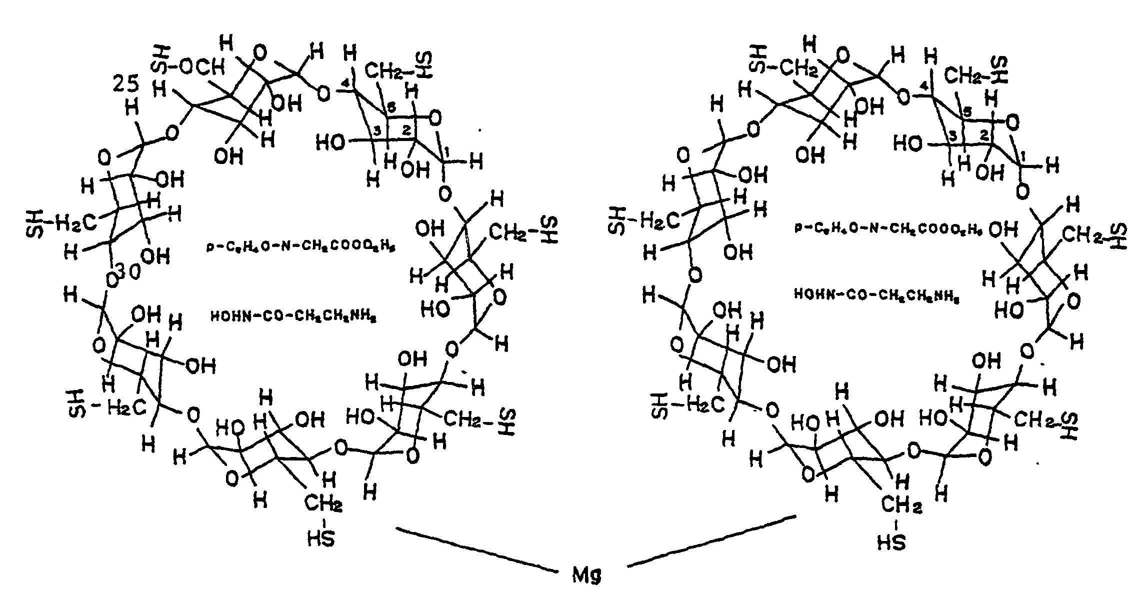

## Claims

1. A process for the production of a chemotherapeutically useful saccharide complex, comprising the steps of:
hydrolysing a saccharide with an enzyme or an acid;
dissolving the saccharide in 2-methoxyethanol together with a further compound, selected from the group consisting of
I (ZnCl₂)-NN-CH₂COOC₂H₅, tent- (CH₃)₃CC₆H₃(OH₂)₂ I(CaCl₂)-NN-CH₂COOC₂H₅, I(CuCl₂)-NHC₆H₃(OH) COOH, HNHNOCNH₄C₆, CaNCOOC₂H₅, I(CaCl₂), CaN-CH₂COOC₂H₅, Na₂ CH₂ COOC₂H₅, Cu(NH₄)₂Cl₄, HONHCONH₂, Mg:NCH₂CH₂Br, 1,4-C₁₀H₆O-NCONHCH₂CH₂-OH, HONHCOCH₂CH₂NH₂, C₅H₄N-CONHNH₂, 1,4-C₁₀H₆O:N-CH₂COOC₂H₅, p-C₆H₄O-NCH₂COOC₂H₅. 6-C₅H₃N₄-N:N-CH₂COOC₂H₅, I (CuCl₂)-NN-CH₂COOC₂H₅, Hg:NCH₂CH₂Br, Cu:NCH₂CH₂Cl Hg:NCH₂CH₂SO₃H, -NN-C₆H₃ (OH) COOH, -N:N-CS-NH(CH₂:CHCH₂), C₄H₂O₃:N-COOC₂H₅ 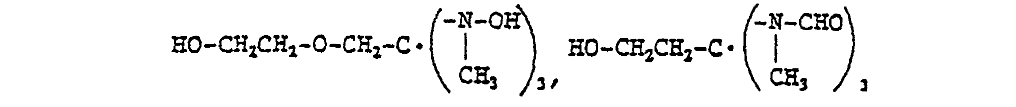 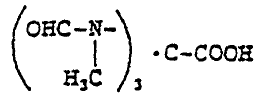 or ester, Schweizer reagent, -N:N-CH₂CH₂SO₃H, -N:N-CH₂COOC₂H₅, Hg:N-CH₂CH₂Br (CH₃)₃CC₆H₃(OCH₃)OH, and (CH₃)₃CC₆H₃(OH)₂:N:N-CH₂COOC₂H₅, and adding a MgSO₄ solution to the solution of the modified saccharide.

2. The process according to claim 1, wherein said hydrolysed saccharide is a saccharide ester or ether or a saccharide with at least one OH-group substituted with -SH, -NH₂, -O-CH₃,-N₃, -F or -BH₄.

3. Process according to any of claims 1 or 2, wherein the saccharide complex comprises at least one compound selected from the group consisting of α-cyclodextrin, β-cyclodextrin and γ-cyclodextrin.

4. Process according to claim 1 or 2, wherein the saccharide complex comprises tert- (CH₃)₃CC₆H₃(OH)₂:N-CH₂COOC₂H₅ and Cu:N-CS-NH(CH₂:CH-CH₂).

5. Process according to claim 1 or 2, wherein the saccharide complex comprises
I(CuCl₂)-NN-C₆H₃(OH)COOH,
C₄H₂N₂O₃-N-CH₂COOC₂H₅ and
Metal fixed pectinase.

6. Process according to claim 1 or 2, wherein the saccharide complex comprises 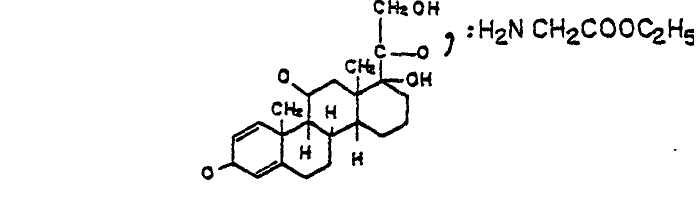 and Sulfatase.

7. Process according to claim 1 or 2, wherein the saccharide complex comprises
Hg:N-CH₂CH₂SO₃H,
Mg:N CH₂CH₂Br and

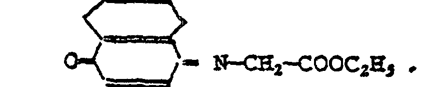

8. Process according to claim 1 or 2, wherein the saccharide complex comprises 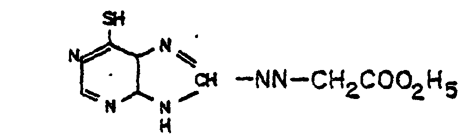 and
-N:N-CS-NH(CH₂:CHCH₂).

9. Process according to claim 3, wherein the saccharide complex comprises
C₁₀H₁₂N₅O₄-N:N-CH₂COOC₂H₅,
Hg:N-CH₂CH₂Br and
-N:N-CH₂COOC₂H₅.

10. Process according to claim 3, wherein the saccharide complex comprises 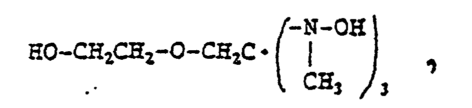 HOHNCOHN -CH₂COOC₂H₅ and
Cu(NH₄)₂ Cl₄-N:N-CH₂COOC₂H₅.

11. Process according to claim 1 or 2, wherein the saccharide complex comprises 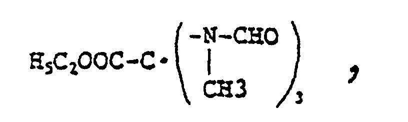 Na₂ CH₂ COOC₂H₅ and
I (ZnCl₂) -NN-CH₂COOC₂H₅,

12. Process according to claim 1 or 2, wherein the saccharide complex comprises
HO-CH₂CH₂-O-CH₂CH₂ (HNCONHON)₃, 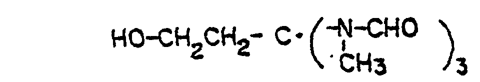 and
Cu(NH₄)₂ Cl₄ -NH-CH₂COOC₂H₅.

13. Process according to claim 1 or 2, wherein the saccharide complex comprises 13. Process according to claim 1 or 2, wherein the complex comprises
tert- (CH₃)₃CC₆H₃(OH₂:N-CH₂COOC₂H₅,
Hg:N-CH₂CH₂-SO₃H and
-NN-C₇H₅O₃.

14. Process according to claim 1 or 2, wherein the saccharide complex comprises
tert- (CH₃)₃CC₆H₃(OH₂:N-CH₂COOC₂H₅,
Hg:N-CH₂CH₂-SO₃H and
-NN-C₇H₅O₃.

15. Process according to claim 3, wherein the saccharide complex comprises
p-C₆H₄O-N-CH₂COOO₂H₅ and
HOHN-CO-CH₂CH₂NH₂.

## Patentansprüche

1. Verfahren zur Herstellung eines chemotherapeutisch einsetzbaren Saccharidkomplexes, umfassend die Schritte:
Hydrolyse eine Saccharids mit einem Enzym oder einer Säure;
Lösen des Saccharids in 2-Methoxyethanol gemeinsam mit einer weiteren Verbindung, die ausgewählt wird aus der aus
I ( ZnCl₂) -NN-CH₂COOC₂H₅, tert- (CH₃)₃CC₆H₃(OH₂)₂ I (CaCl₂) -NN-CH₂COOC₂H₅, I (CuCl₂)-NHC₆H₃(OH)COOH, HNHNOCNH₄C₆, CaNCOOC₂H₅, I(CaCl₂), CaN-CH₂COOC₂H₅, Na₂ CH₂ COOC₂H₅, Cu (NH₄)₂Cl₄, HONHCONH₂, Mg: NCH₂CH₂Br, 1,4-C₁₀H₆O-NCONHCH₂CH₂-OH, HONHCOCH₂CH₂NH₂, C₅H₄N-CONHNH₂, 1,4-C₁₀H₆O:M-CH₂COOC₂H₅, p-C₆H₄O-NCH₂COOC₂H₅. 6 -C₅H₃N₄-N:N-CH₂COOC₂H₅, I(CuCl₂)-NN-CH₂COOC₂H₅, Hg:NCH₂CH₂Br, Cu:NCH₂CH₂Cl Hg:NCH₂CH₂SO₃H, -NN-C₆H₃ (OH)COOH, -N:N-CS-NH(CH₂:CHCH₂), C₄H₂N₂O₃:N-COOC₂H₅ 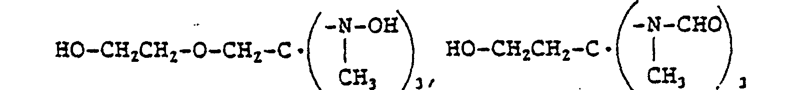 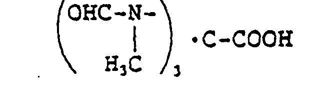 oder Ester, Schweizer-Reagens, -N:N-CH₂CH₂SO₃H, -N:N-CH₂COOC₂H₅, Hg: N-CH₂CH₂Br (CH₃)₃CC₆R₃(OCH₃)OH, and (CH₃)₃CC₆H₃ (OH) ₂: N : N-CH₂COOC₂H₅
bestehenden Gruppe und Zugabe einer MgSO₄-Lösung zu der Lösung des modifizierten Saccharids.

2. Verfahren nach Anspruch 1, wobei das hydolisierte Saccharid ein Saccharidester oder -ether oder ein Saccharid mit mindestens einer OH-Gruppe ist, die substituiert ist durch -SH, -NH₂, -O-CH₃, -N₃, -F oder -BH₄.

3. Verfahren nach einem der Ansprüche 1 oder 2, wobei der Saccharidkomplex mindestens eine Verbindung umfasst, die ausgewählt wird aus der aus α-Cyclodextrin, β-Cyclodextrin und γ-Cyclodextrin bestehenden Gruppe.

4. Verfahren nach Anspruch 1 oder 2, wobei der Saccharidkomplex
Tert-(CH₃)₃CC₆H₃(OH)₂:N-CH₂COOC₂H₅
und Cu:N-CS-NH(CH₂:CH-CH₂)
umfasst.

5. Verfahren nach Anspruch 1 oder 2, wobei der Saccharidkomplex
I(CuCl₂)-NN-C₆H₃(OH) COOH,
C₄H₂N₂O₃-N-CH₂COOC₂H₅
und metallfixierte Pectinase
umfasst.

6. Verfahren nach Anspruch 1 oder 2, wobei der Saccharidkomplex 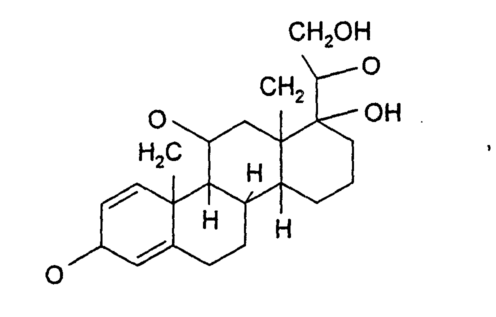 H₂NCH₂COOC₂H₅
und Sulfatase
umfasst.

7. Verfahren nach Anspruch 1 oder 2, wobei der Saccharidkomplex
Hg : N-CH₂CH₂SO₃H,
Mg : N CH₂CH₂Br und 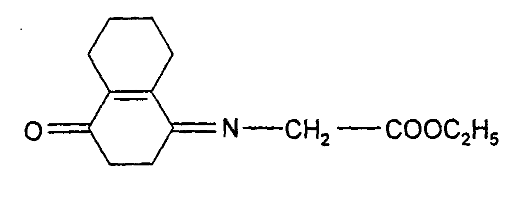 umfasst.

8. Verfahren noch Anspruch 1 oder 2, wobei der Saccharidkomplex 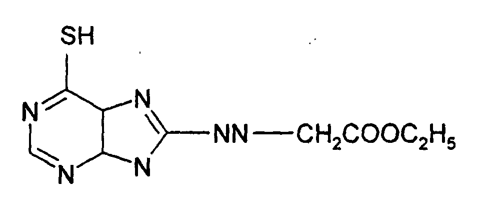 und -N:N-CS-NH(CH₂:CHCH₂)
umfaßt.

9. Verfahren nach Anspruch 3, wobei der Saccharidkomplex
C10H12N5O4-N-CH₂COOC₂H₅,
Hg:N-CH₂CH₂Br
und -N:N-CH₂COOC₂H₅
umfasst.

10. Verfahren nach Anspruch 3, wobei der Saccharidkomplex 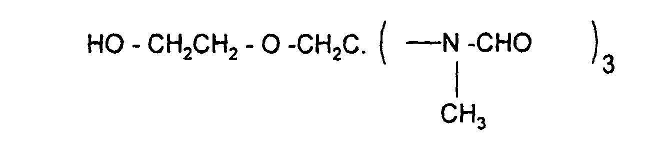 HOHNCOHN -CH₂COOC₂H₅
und Cu(NH₄)₂ Cl₄-N:N-CH₂COOC₂H₅
umfasst.

11. Verfahren nach Anspruch 1 oder 2, wobei der Saccharidkomplex 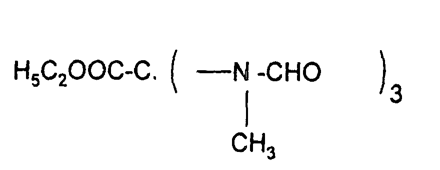 Na₂CH₂COOC₂H₅
und I(ZnCl₂)-NN-CH₂COOC₂H₅
unfasst.

12. Verfahren nach Anspruch 1 oder 2, wobei der Saccharidkomplex
HO-CH₂CH₂-O-CH₂CH₂(HNCONHON)₃, 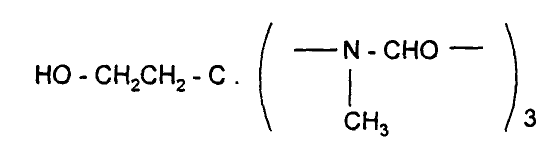 und Cu(NH₄)₂ Cl₄-NH-CH₂COOC₂H₅
umfaßt.

13. Verfahren nach Anspruch 1 oder 2, wobei der Saccharidkomplex
Tert-(CH₃)₃CC₆H₃(OH)₂:N-CH₂COOC₂H₅
Hg:N-CH₂CH₂SO₃H
und -NN-C₇H₅O₃
umfasst.

14. Verfahren nach Anspruch 1 oder 2, wobei der Saccharidkomplex
Tert-(CH₃)₃CC₆H₃(OH)₂:N-CH₂COOC₂H₅
Hg:N-CH₂CH₂SO₃H
und -NN-C₇H₅O₃
umfasst.

15. Verfahren nach Anspruch 3, wobei der Saccharidkomplex
p-C₆H₄O-N-CH₂COOC₂H₅
und HOHN-CO-CH₂CH₂NH₂
umfaßt.

## Revendications

1. Procédé de production d'un complexe de saccharide utile du point de vue chimiothérapeutique, comprenant les étapes consistant :
• à hydrolyser un satcharide avec une enzyme ou un acide ;
• à dissoudre le saccharide dans du 2-méthoxyéthanol avec un autre composé, choisi dans l'ensemble consistant en :
I(ZnCl₂)-NN-CH₂COOC₂H₅, tert-H₃(OH)COOH, HNHNOCNH₄C₆, CaN-COOC₂H₅, I(CaCl₂), CaN-CH₂COOC₂H₅, Na₂CH₂COOC₂H₅, Cu(NH₄)₂Cl₄, HONHCONH₂, Mg:NCH₂CH₂Br, 1,4-C₁₀H₆O-NCONHCH₂CH₂-OH, HON-HCOCH₂CH₂NH₂, C₅H₄-CONHNH₂, 1,4-C₁₀H₆O:N-CH₂COOC₂H₅, p-C₆H₄-NCH₂COOC₂H₅, 6-C₅H₃N₄-N:N-CH₂COOC₂H₅, I(CuCl₂)-NN-CH₂COO-C₂H₅, Hg:NCH₂CH₂Br, Cu:NCH₂CH₂Cl, Hg:NCH₂CH₂SO₃H, NN-C₆H₃(OH)-COOH, -N:N-CS-NH(CH₂:CHCH₂), C₄H₂N₂O₃:N-COOC₂H₅, 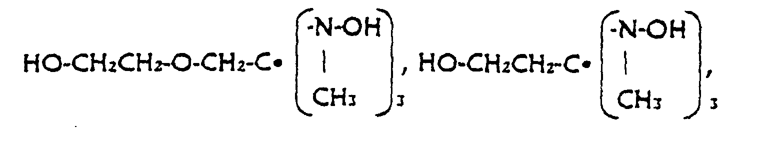 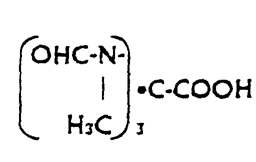 ou un ester, un réactif de Schweizer, -N:N-CH₂CH₂S-O₃H, -N:N-CH₂COOC₂H₅, Hg:N-CH₂CH₂Br, (CH₃)₃CCH₃(OCH₃)OH, et (CH₃)₃CC₆H₅(OH)₂:N:N-CH₂COOC₂H₅, et à ajouter une solution de MgSO₄ à la solution du saccharide modifié.

2. Procédé selon la revendication 1, dans lequel ledit saccharide hydrolysé est un ester ou un éther de saccharide ou un saccharide avec au moins un groupe OH substitué par -SH₂, -NH₂, -O-CH₃, -N₃, F ou -BH₄.

3. Procédé selon l'une quelconque des revendications 1 ou 2, dans lequel le complexe de saccharide comprend au moins un composé choisi dans l'ensemble constitué d'α-cyclodextrine, β-cyclodextrine et γ-cyclodextrine.

4. Procédé selon la revendication 1 ou 2, dans lequel le complexe de saccharide comprend tert.-(CH₃)₃CC₆H₃(OH₂)₂:N-CH₂COOC₂H₅, et Cu:N-CS-NH-(CH₂:CH-CH₂).

5. Procédé selon la revendication 1 ou 2, dans lequel le complexe de saccharide comprend I(CuCl₂)-N-C₆H₃(OH)COOH, C₄H₂N₂O₃-N-CH₂COOC₂H₅ et une pectinase fixée à un métal.

6. Procédé selon la revendication 1 ou 2, dans lequel le complexe de saccharide comprend: 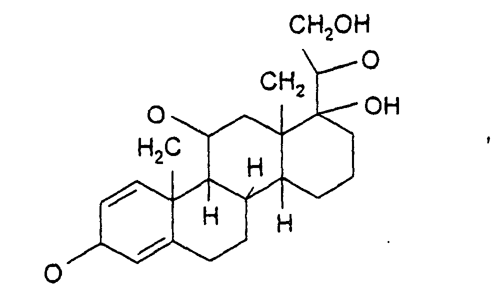 :H₂NCH₂COOC₂H₅ et une sulfatase.

7. Procédé selon la revendication 1 ou 2, dans lequel le complexe de saccharide comprend Hg:N-CH₂CH₂SO₃H, Mg:N CH₂CH₂Br et

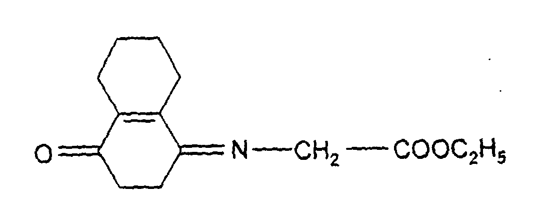

8. Procédé selon la revendication 1 ou 2, dans lequel le complexe de saccharide comprend : 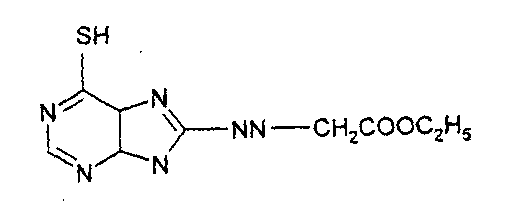 et-N:N-CS-NH(CH₂:CHCH₂).

9. Procédé selon la revendication 3, dans lequel le complexe de saccharide comprend C₁₀H₁₂N₅O₄-N:N-CH₂COOC₂H₅, Hg:NCH₂CH₂Br et -N:N-CH₂C-OOC₂H₅.

10. Procédé selon la revendication 3, dans lequel le complexe de sacchande comprend: 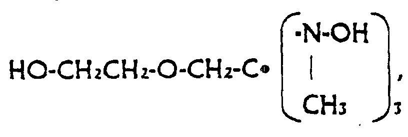 HOHNCOCH-CH₂COOC₂H₅ et Cu- (NH₄)₂Cl₄-N:N-CH₂COOC₂H₅.

11. Procédé selon la revendication 1 ou 2, dans lequel le complexe de saccharide comprend : 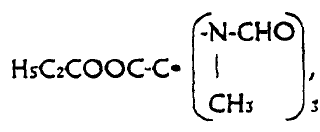 Na₂CH₂COOC₂H₅ et I(ZnCl₂)-NN-CH₂COO- C2H5.

12. Procédé selon la revendication 1 ou 2, dans lequel le complexe de saccharide comprend :
HO-CH₂CH₂-O-CH₂CH₂(HNCONHON)₃, 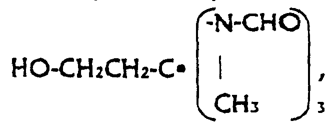 et Cu(NH₄)₂Cl₄-NH-CH₂COOC₂H₅.

13. Procédé selon la revendication 1 ou 2, dans lequel le complexe de saccharide comprend tert.-(CH₃)₃CC₆H₃(OH)₂:N-CH₂COO₂H₅, Hg:N-CH₂CH₂-SO₃H et NN-C₇H₅O₃.

14. Procédé selon la revendication 1 ou 2, dans lequel le complexe de saccharide comprend tert.-(CH₃)₃CC₆H₃(OH)₂:N:N-CH₂COO₂H₅, Hg:N-CH₂CH₂-SO₃H et NN-C₇H₅O₃.

15. Procédé selon la revendication 3, dans lequel le complexe de saccharide comprend p-C₆H₆O-N-CH₂COOC₂H₅ et HONH-CO-CH₂CH₂NH₂.
